(19) Europäisches Patentamt
European Patent Office
Office européen des brevets

(11) Publication number: **0 046 626**
**B1**

(12) # EUROPEAN PATENT SPECIFICATION

(45) Date of publication of patent specification: **08.02.84**

(51) Int. Cl.³: **C 07 C 143/828**

(21) Application number: **81201218.5**

(22) Date of filing: **29.05.79**

(60) Publication number of the earlier application in accordance with Art. 76 EPC: **0007687**

(54) **Substituted benzenesulfonyl isocyanates and preparation thereof.**

(30) Priority: **30.05.78 US 910965**
**30.11.78 US 965070**
**01.03.79 US 15341**
**13.04.79 US 29281**

(43) Date of publication of application:
**03.03.82 Bulletin 82/9**

(45) Publication of the grant of the patent:
**08.02.84 Bulletin 84/6**

(84) Designated Contracting States:
**BE CH DE FR GB IT LU NL SE**

(56) References cited:

**ARZNEIMITTEL-FORSCHUNG, vol. 22, no. 12a, December 1972, Edition Cantor KG, AULENDORF (DE), F. BIGLER et al.: "Über den Stoffwechsel von Glibornurid beim Menschen" pages 2191-2198**

**The file contains technical information submitted after the application was filed and not included in this specification**

(73) Proprietor: **E.I. DU PONT DE NEMOURS AND COMPANY**
**Legal Department 1007 Market Street**
**Wilmington Delaware 19898 (US)**

(72) Inventor: **Levitt, George**
**3218 Romilly Road**
**Cardiff Wilmington Delaware 19810 (US)**

(74) Representative: **Hildyard, Edward Martin et al,**
**Frank B. Dehn & Co. European Patent Attorneys**
**Imperial House 15-19 Kingsway**
**London WC2B 6UZ (GB)**

**EP 0 046 626 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Substituted benzenesulfonyl isocyanates and preparation thereof

This invention concerns novel substituted benzene-sulfonyl isocyanates and a process for their preparation. The novel compounds all have a carboxylic ester substituent in the *ortho*-position. They may be represented by the general formula:

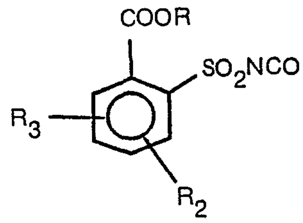

(I)

wherein
R is $C_1$—$C_{12}$ alkyl; $C_3$—$C_{10}$ alkenyl; $C_2$—$C_6$ alkyl substituted with one to four substituents selected from 0—3 atoms of F, Cl, Br, 0—2 methoxy groups; $C_3$—$C_6$ alkenyl substituted with 1—3 atoms of F, Cl, Br; $C_5$—$C_8$ cycloalkyl; $C_5$—$C_8$ cycloalkenyl; $C_5$—$C_6$ cycloalkyl substituted with any of one to four methyl groups, methoxy, alkyl substituents of $C_2$—$C_4$, F, Cl or Br; $C_4$—$C_{10}$ cycloalkylalkyl; $C_4$—$C_8$ cycloalkylalkyl with 1—2 $CH_3$; —$CH_2CH_2OR_7$; $CH_2CH_2CH_2OR_7$;

$CH—CH_2OR_7$
|
$CH_3$

where $R_7$ is —$CH_2CH_3$, $CH(CH_3)_2$, phenyl, —$CH_2CH_2Cl$, —$CH_2CCl_3$; $-(CH_2CH_2O)_{n'}R_8$;

$-(CHCH_2O)_{n'}R_8$
|
$CH_3$

where $R_8$ is $CH_3$, —$CH_2CH_3$; —$CH(CH_3)_2$, phenyl, —$CH_2CH_2Cl$, —$CH_2CCl_3$, and n' is 2 or 3;
$R_2$ is H, Cl, Br, F, $C_1$—$C_3$ alkyl, —$NO_2$, —$OCH_3$, —$SCH_3$, $CF_3$, $SO_2CH_3$, $N(CH_3)_2$, CN;
$R_3$ is H, Cl, Br or $CH_3$.

The compounds of general formula (I) are valuable intermediates for the preparation of the N-(heterocyclicaminocarbonyl)arylsulfonamides disclosed in our EP—A—7687, to which the reader is referred for further information. The said arylsulfonamides possess potent herbicidal and plant growth regulant activities.

The following preferences for certain substituents in the compounds of general formula (I) are based primarily on the activity of the N-(heterocyclicaminocarbonyl)arylsulfonamides obtainable from them:

1) A compound of general formula (I) wherein $R_3$ is H and is para to the sulfonyl group.

2) A compound of preferred 1) wherein R is $C_1$—$C_6$ alkyl; $C_2$—$C_4$ alkyl substituted with one to four substituents selected from 0—3 atoms of F or Cl and 0—2 methoxy groups; $C_3$—$C_6$ alkenyl; $C_3$—$C_4$ alkenyl substituted with 1—3 Cl atoms; $C_5$—$C_6$ cycloalkyl; $C_5$—$C_6$ cycloalkenyl; $C_5$—$C_6$ cycloalkyl substituted with any one of one to four methyl groups, methoxy, —$C_2H_5$ and Cl; $C_4$—$C_7$ cycloalkylalkyl; —$CH_2CH_2OR_7$; —$CH_2CH_2CH_2OR_7$;

—$CH$—$CH_2OR_7$
|
$CH_3$

where $R_7$ is as previously defined; $-(CH_2CH_2O)_2R_8$ or

$-(CHCH_2O)_2R_8$
|
$CH_3$

where $R_8$ is $CH_3$, —$CH_2CH_3$, —$CH(CH_3)_2$ or —$CH_2CH_2Cl$.

3) A compound of preferred 2) wherein $R_2$ is H, Cl or $CH_3$ and R is $C_1$—$C_4$ alkyl; $C_3$—$C_4$ alkenyl; $C_2$—$C_3$ alkyl substituted with —$OCH_3$ or Cl; $C_3$-alkenyl substituted with 1—3 Cl atoms; $C_5$—$C_6$ cycloalkyl; cyclohexenyl; cyclohexyl substituted with 1—3 methyl groups; —$CH_2CH_2OR_7$ where $R_7$ is —$CH_2H_5$, —$CH(CH_3)_2$, phenyl or —$CH_2CH_2Cl$; or

$$\underset{\displaystyle CH_3}{\overset{}{-CHCH_2OC_2H_5.}}$$

4) A compound of preferred 3) wherein $R_2$ and $R_3$ are both H.

5) A compound of preferred 2, 3 or 4 wherein R is $C_1$—$C_4$ alkyl, $C_2$—$C_3$ alkyl substituted with Cl; $C_3$—$C_4$ alkenyl; $—CH_2CH_2OCH_3$;

$$\underset{\displaystyle CH_3}{-CHCH_2OCH_3};\ -CH_2CH_2OC_2H_5;\ \underset{\displaystyle CH_3}{-CHCH_2OC_2H_5};\ \text{or}\ -CH_2CH_2CH_2OC_2H_5.$$

Compounds of particular interest include methyl 2-(isocyanatosulfonyl)benzoate, isopropyl 2-(isocyanatosulfonyl)benzoate, allyl 2-(isocyanatosulfonyl)benzoate, 1-(2-chloroethyl) 2-(isocyanatosulfonyl)benzoate and propyl 2-(isocyanatosulfonyl)benzoate.

The compounds of general formula (I) can be made by reacting a corresponding sulfonamide of general formula

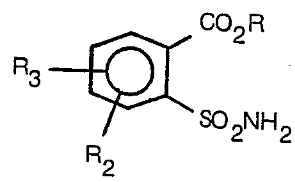

(II)

wherein R, $R_2$ and $R_3$ are as hereinbefore defined, with phosgene in the presence of a catalytic amount of 1,4-diaza[2,2,2]bicyclooctane in an inert solvent e.g. as illustrated below:

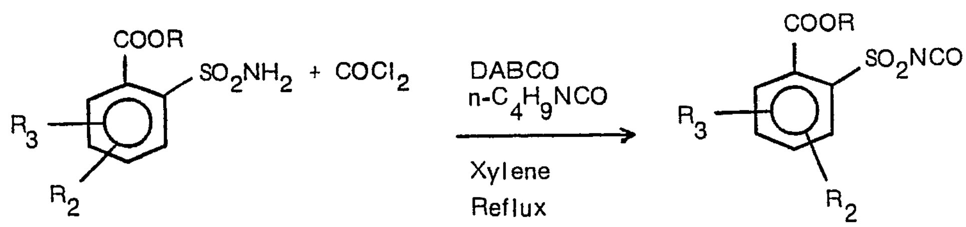


(II) (I)

A mixture of the appropriate sulfonamide, e.g. an *o*-alkoxycarbonyl benzenesulfonamide II such as the methyl ester, which is known in the art, an alkyl isocyanate such as butyl isocyanate and a catalytic amount of 1,4-diaza[2,2,2]bicyclooctane (DABCO) in xylene or other inert solvent of sufficiently high boiling point (e.g. >135°) is heated to approximately 135°. Phosgene is added to the mixture until an excess of phosgene is present as indicated by a drop in the boiling point. (The mixture is heated further to drive off the excess phosgene). After the mixture is cooled and filtered to remove a small amount of insoluble by-products, the solvent and alkyl isocyanate are distilled off *in-vacuo* leaving a residue which is the crude sulfonyl isocyanate I.

The following Examples are given by way of illustration only. All temperatures are in °C.

Example 1

*Methyl 2-(isocyanatosulfonyl)benzoate*

A stirred mixture containing 157 g of methyl 2-sulfamoylbenzoate, 73 g of butyl isocyanate 0.3 g of 1,4-diazabicyclo[2,2,2]octane and 1.0 l of xylene was heated to reflux for one half hour. Phosgene gas was then passed into the system under a dry ice reflux condenser allowing the reaction temperature to drop to 120°. This addition was contained until the reflux temperature remained at 120° without further phosgene addition. The temperature of the reaction mixture was then raised to 136° (by removal of the dry ice reflux condenser) after which it was cooled to room temperature and filtered. Evaporation of the filtrate yielded the desired crude sulfonyl isocyanate which could be purified by distillation at 132—138°C under 1.0 to 1.1 mm of mercury pressure. The product is extremely reactive with water so contact with moisture should be scrupulously avoided.

Example 2

*Isopropyl 2-(isocyanatosulfonyl)benzoate*

To 60.7 g (.25 mole) of isopropyl 2-sulfamoylbenzoate in 300 ml dry (molecular sieves) xylenes was added 25.0 g (.25 mole) N-butyl isocyanate and .1 g 1,4-diazabicyclo[2,2,2]octane. The mixture was heated to reflux temperature and phosgene was slowly bubbled through the solution for 2 hours.

An infrared spectrum of the reaction mixture indicated formation of the desired sulfonylisocyanate (2250 $cm^{-1}$). The resulting cloudy solution was cooled to room temperature and decanted from a small amount of solid impurity. Evaporation of the resulting clear solution yielded the desired crude sulfonyl isocyanate, which was used in subsequent steps without further purification.

Analogously to these Examples one may prepare the isocyanates listed in Table I.

TABLE I

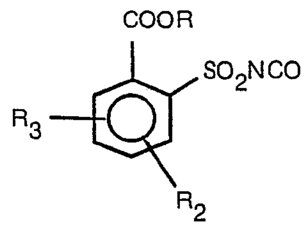

| R | $R_2$ | $R_3$ |
|---|---|---|
| $C_2H_5$ | H | H |
| *n*-$C_3H_7$ | H | H |
| $CHCH_2CH_3$<br>$CH_3$ | H | H |
| $CH_2CH_2Cl$ | H | H |
| $(CH_2)_9CH_3$ | H | H |
| 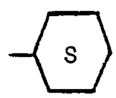 | H | H |
| $CH_3$ | 4-Cl | H |
| *n*-$C_4H_9$ | H | H |
| $CHCH_2Cl$<br>$CH_3$ | H | H |
| $CH_2CH = CH_2$ | H | H |
| $CH(CH_2)_2CH = CH_2$<br>$CH_3$ | H | H |
| $CH_3$ | 4-Cl | 5-Cl |
| $CH_3$ | 4-F | H |
| $CH_2CH(CH_3)_2$ | H | H |
| $(CH_2)_4CH_3$ | H | H |
| $CHCH_2CH_3$<br>$CH_2CH_3$ | H | H |
| $CH_2CH_2OCH_2CH_3$ | H | H |
| $CH_2CH_2CH(CH_3)_2$ | H | H |
| $(CH_2)_5CH_3$ | H | H |

TABLE I (continued)

| R | $R_2$ | $R_3$ |
|---|---|---|
| $(CH_2CH_2O)_2C_2H_4Cl$ | H | H |
| $CH_2CH_2OC_2H_4Cl$ | H | H |
| $CH_2CH_2O$— 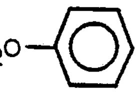 | H | H |
| 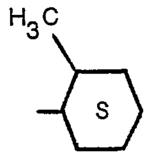 | H | H |

## Claims

1. A compound having the general formula

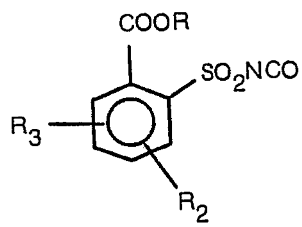

(I)

wherein
R is $C_1$—$C_{12}$ alkyl; $C_3$—$C_{10}$ alkenyl; $C_2$—$C_6$ alkyl substituted with one to four substituents selected from 0—3 atoms of F, Cl, Br, 0—2 methoxy groups; $C_3$—$C_6$ alkenyl substituted with 1—3 atoms of F, Cl, Br; $C_5$—$C_8$ cycloalkyl; $C_5$—$C_8$ cycloalkenyl; $C_5$—$C_6$ cycloalkyl substituted with any of one to four methyl groups, methoxy, alkyl substituents of $C_2$—$C_4$, F, Cl or Br; $C_4$—$C_{10}$ cycloalkylalkyl; $C_4$—$C_8$ cycloalkylalkyl with 1—2 $CH_3$; —$CH_2CH_2OR_7$; $CH_2CH_2CH_2OR_7$;

$$\underset{\displaystyle CH_3}{CH}—CH_2OR_7$$

where $R_7$ is —$CH_2CH_3$, $CH(CH_3)_2$, phenyl, —$CH_2CH_2Cl$, —$CH_2CCl_3$; $-(CH_2CH_2O)_{n'}R_8$;

$$-(\underset{\displaystyle CH_3}{CH}CH_2O)_{n'}R_8$$

where $R_8$ is $CH_3$, —$CH_2CH_3$; —$CH(CH_3)_2$, phenyl, —$CH_2CH_2Cl$, —$CH_2CCl_3$, and n' is 2 or 3;
$R_2$ is H, Cl, Br, F, $C_1$—$C_3$ alkyl, —$NO_2$, —$OCH_3$, —$SCH_3$, $CF_3$, $SO_2CH_3$, $N(CH_3)_2$, CN;
$R_3$ is H, Cl, Br or $CH_3$.

2. A compound of claim 1 wherein $R_3$ is H and is para to the sulfonyl group.

3. A compound of claim 2 wherein R is $C_1$—$C_6$ alkyl; $C_2$—$C_4$ alkyl substituted with one to four substituents selected from 0—3 atoms of F or Cl and 0—2 methoxy groups; $C_3$—$C_6$ alkenyl; $C_3$—$C_4$ alkenyl substituted with 1—3 Cl atoms; $C_5$—$C_6$ cycloalkyl; $C_5$—$C_6$ cycloalkenyl; $C_5$—$C_6$ cycloalkyl substituted with any of one to four methyl groups, methoxy, —$C_2H_5$ and Cl; $C_4$—$C_7$ cycloalkylalkyl; —$CH_2CH_2OR_7$; —$CH_2CH_2CH_2OR_7$;

$$—\underset{\displaystyle CH_3}{CH}—CH_2OR_7$$

where $R_7$ is as defined in claim 1; $-(CH_2CH_2O)_2R_8$ or

$$-(\underset{CH_3}{\underset{|}{CH}}CH_2O)_2R_8$$

where $R_8$ is $CH_3$, $-CH_2CH_3$, $-CH(CH_3)_2$ or $-CH_2CH_2Cl$.

4. A compound of claim 3 wherein $R_2$ is H, Cl or $CH_3$ and R is $C_1$—$C_4$ alkyl; $C_3$—$C_4$ alkenyl; $C_2$—$C_3$ alkyl substituted with $-OCH_3$ or Cl; $C_3$-alkenyl substituted with 1—3 Cl atoms; $C_5$—$C_6$ cycloalklyl; cyclohexenyl; cyclohexyl substituted with 1—3 methyl groups; $-CH_2CH_2OR_7$ where $R_7$ is $-C_2H_5$, $-CH(CH_3)_2$, phenyl or $-CH_2CH_2Cl$; or

$$-\underset{CH_3}{\underset{|}{CH}}CH_2OC_2H_5.$$

5. A compound of claim 4 wherein $R_2$ and $R_3$ are both H.

6. A compound of claim 3, 4 or 5 wherein R is $C_1$—$C_4$ alkyl; $C_2$—$C_3$ alkyl substituted with Cl; $C_3$—$C_4$ alkenyl; $-CH_2CH_2OCH_3$;

$$-\underset{CH_3}{\underset{|}{CH}}CH_2OCH_3;\ -CH_2CH_2OC_2H_5;\ -\underset{CH_3}{\underset{|}{CH}}CH_2OC_2H_5;\ \text{or}\ -CH_2CH_2CH_2OC_2H_5.$$

7. Methyl 2-(isocyanatosulfonyl)benzoate.

8. Isopropyl 2-(isocyanatosulfonyl)benzoate.

9. Allyl 2-(isocyanatosulfonyl)benzoate.

10. 1-(2-chloroethyl) 2-(isocyanatosulfonyl)benzoate.

11. Propyl 2-(isocyanatosulfonyl)benzoate.

12. A process for preparing a compound of claim 1 which comprises reacting a compound of general formula

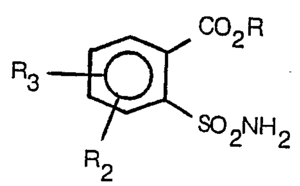

(II)

wherein R, $R_2$ and $R_3$ are as defined in claim 1, with phosgene in the presence of a catalytic amount of an alkyl isocyanate and a catalytic amount of 1,4-diaza[2,2,2]bicyclooctane in an inert solvent.

**Patentansprüche**

1. Verbindung mit der allgemeinen Formel I

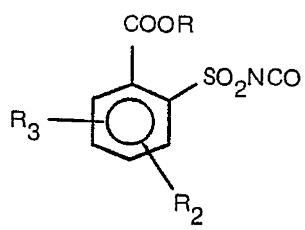

(I)

worin R $C_1$—$C_{12}$ Alkyl; $C_3$—$C_{10}$ Alkenyl; $C_2$—$C_6$ Alkyl substituiert mit einem bis vier Substituenten, ausgewählt aus 0—3 Atomen von F, Cl, Br, 0—2 Methoxygruppen; $C_3$—$C_6$ Alkenyl substituiert mit 1—3 Atomen von F, Cl, Br; $C_5$—$C_8$ Cycloalkyl; $C_5$—$C_8$ Cycloalkenyl; $C_5$—$C_6$ Cycloalkyl substituiert mit beliebigen von 1 bis 4 Methylgruppen, Methoxy, Alkylsubstituenten mit $C_2$—$C_4$, F, Cl, oder Br; $C_4$—$C_{10}$ Cycloalkylalkyl; $C_4$—$C_8$ Cycloalkylalkyl mit 1—2 $CH_3$; $-CH_2CH_2OR_7$; $CH_2CH_2CH_2OR_7$;

$$\underset{\displaystyle CH_3}{CH}—CH_2OR_7$$

worin $R_7$ die Bedeutung hat von $—CH_2CH_3$, $CH(CH_3)_2$, Phenyl, $—CH_2CH_2Cl$, $—CH_2CCl_3$; $-(CH_2CH_2O)_{n'}-R_8$;

$$-(\underset{\displaystyle CH_3}{CH}CH_2O)_{n'}-R_8$$

worin $R_8$ die Bedeutung hat von $CH_3$, $—CH_2CH_3$, $—CH(CH_3)_2$, Phenyl, $—CH_2CH_2Cl$, $—CH_2CCl_3$, und n' 2 oder 3 bedeutet; ist

$R_2$ H, Cl, Br, F, $C_1—C_3$ Alkyl, $—NO_2$, $—OCH_3$, $—SCH_3$, $CF_3$, $SO_2CH_3$, $N(CH_3)_2$, CN ist;

$R_3$ H, Cl, Br oder $CH_3$ ist.

2. Verbindung nach Anspruch 1, worin $R_3$ H ist und in para-Stellung zur Sulfonylgruppe steht.

3. Verbindung nach Anspruch 2, worin R $C_1—C_6$ Alkyl; $C_2—C_4$ Alkyl substituiert mit einem bis vier Substituenten, ausgewählt aus 0—3 Atomen von F oder Cl und 0—2 Methoxygruppen; $C_3—C_6$ Alkenyl; $C_3—C_4$ Alkenyl; substituiert mit 1—3 Cl Atomen; $C_5—C_6$ Cycloalkyl; $C_5—C_6$ Cycloalkenyl; $C_5—C_6$ Cycloalkyl substituiert mit beliebigen von 1 bis 4 Methylgruppen, Methoxy, $—C_2H_5$ und Cl; $C_4—C_7$ Cycloalkylalkyl; $—CH_2CH_2OR_7$; $—CH_2CH_2CH_2OR_7$;

$$—\underset{\displaystyle CH_3}{CH}—CH_2OR_7$$

worin $R_7$ wie in Anspruch 1 definiert ist; $CH_3$; $-(CH_2CH_2O)_2-R_8$ oder

$$-(\underset{\displaystyle CH_3}{CH}CH_2O)_2-R_8$$

worin $R_8CH_3$, $—CH_2CH_3$, $—CH(CH_3)_2$ oder $—CH_2CH_2Cl$ ist; bedeutet.

4. Verbindung nach Anspruch 3, worin $R_2$, H, Cl oder $CH_3$ ist und R $C_1—C_4$ Alkyl; $C_3—C_4$ Alkenyl; $C_2—C_3$ Alkyl substituiert mit $—OCH_3$ oder Cl; $C_3$-Alkenyl substituiert mit 1—3 Cl Atomen; $C_5—C_6$ Cycloalkyl; Cyclohexenyl; Cyclohexyl substituiert mit 1—3 Methylgruppen; $—CH_2CH_2OR_7$ worin $R_7$ die Bedeutung hat von $—C_2H_5$, $—CH(CH_3)_2$, Phenyl oder $—CH_2CH_2Cl$; oder

$$—\underset{\displaystyle CH_3}{CH}CH_2OC_2H_5;\text{ ist.}$$

5. Verbindung nach Anspruch 4, worin $R_2$ und $R_3$ beide H sind.

6. Verbindung nach Anspruch 3, 4 oder 5, worin R $C_1—C_4$ Alkyl, $C_2—C_3$ alkyl substituiert mit Cl; $C_3—C_4$ Alkenyl; $—CH_2CH_2OCH_3$;

$$—\underset{\displaystyle CH_3}{CH}CH_2OCH_3;\ —CH_2CH_2OC_2H_5;\ —\underset{\displaystyle CH_3}{CH}CH_2OC_2H_5;$$

oder $—CH_2CH_2CH_2OC_2H_5$; ist.

7. Methyl-2-(isocyanatosulfonyl)-benzoat.

8. Isopropyl-2-(isocyanatosulfonyl)-benzoat.

9. Alyl-2-(isocyanatosulfonyl)-benzoat.

10. 1-(2-Chlorethyl)-2-(isocyanatosulfonyl)-benzoat.

11. Propyl-2-(isocyanatosulfonyl)-benzoat.

12. Verfahren zur Herstellung einer Verbindung nach Anspruch 1, durch Reaktion einer Verbindung der allgemeinen Formel

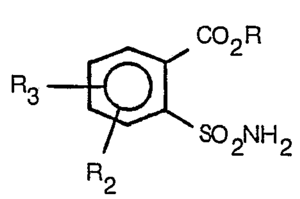

worin R, $R_2$ und $R_3$ wie in Anspruch 1 definiert sind, mit Phosgen in Anwesenheit einer katalytischen Menge eines Alkyl-isocyanats und einer katalytischen Menge von 1,4-Diaza[2,2,2]bicyclooctan in einem inerten Lösungsmittel.

**Revendications**

1. Un composé ayant la formule générale

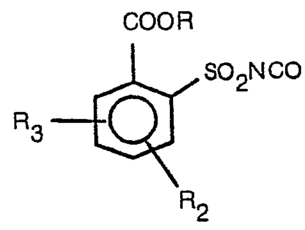

(I)

dans laquelle

R est un radical alcoyle en $C_1$—$C_{12}$; alcényle en $C_3$—$C_{10}$; alcoyle en $C_2$—$C_6$ substitué par un à quatre substituants choisis parmi 0—3 atomes de F, Cl, Br, 0—2 groupes méthoxy; alcényle en $C_3$—$C_6$ substitué par 1—3 atomes de F, Cl, Br; cycloalcoyle en $C_5$—$C_8$; cycloalcényle en $C_5$—$C_8$; cycloalcoyle en $C_5$—$C_6$ substitué par un à quatre substituants quelconques choisis parmi les substituants méthyle, méthoxy, alcoyle en $C_2$—$C_4$, F, Cl ou Br; cycloalcoylalcoyle en $C_4$—$C_{10}$; cycloalcoylalcoyle en $C_4$—$C_8$ avec 1—2 $CH_3$; —$CH_2CH_2OR_7$; $CH_2CH_2CH_2OR_7$;

$$\underset{\displaystyle CH_3}{CH}—CH_2OR_7$$

où $R_7$ est —$CH_2CH_3$, $CH(CH_3)_2$, un radical phényle, —$CH_2CH_2Cl$, —$CH_2CCl_3$;$-\!\!(CH_2CH_2O)_{n'}$ $R_8$;

$$-\!\!(\underset{\displaystyle CH_3}{CH}CH_2O)_{n'}R_8$$

où $R_8$ est $CH_3$, —$CH_2CH_3$ —$CH(CH_3)_2$, un radical phényle, —$CH_2CH_2Cl$, —$CH_2CCl_3$, et n' est 2 ou 3; $R_2$ est H, Cl, Br, F, un radical alcoyle en $C_1$—$C_3$, —$NO_2$, —$OCH_3$, —$SCH_3$, $CF_3$, $SO_2CH_3$, $N(CH_3)_2$, CN; $R_3$ est H, Cl, Br ou $CH_3$.

2. Un composé selon la revendication 1, dans lequel $R_3$ est H et est en position para par rapport au groupe sulfonyle.

3. Un composé selon la revendication 2, dans lequel R est un radical alcoyle en $C_1$—$C_6$; alcoyle en $C_2$—$C_4$ substitué par 1 à 4 substituants choisis parmi 0—3 atomes de F ou Cl et 0—2 groupes méthoxy; alcényle en $C_3$—$C_6$; alcényle en $C_3$—$C_4$ substitué par 1—3 atomes de Cl; cycloalcoyle en $C_5$—$C_6$; cycloalcényle en $C_5$—$C_6$; cycloalcoyle en $C_5$—$C_6$ substitué par un à quatre substituants quelconques choisis parmi les groupes méthyle, méthoxy, —$C_2H_5$ et Cl; cycloalcoylalcoyle en $C_4$—$C_7$; —$CH_2CH_2OR_7$; —$CH_2CH_2CH_2OR_7$;

$$—\underset{\displaystyle CH_3}{CH}—CH_2OR_7 \text{ où}$$

$R_7$ est tel que défini dans la revendication 1;$-\!\!(CH_2CH_2O)_2R_8$ ou

$$-\!\!(\underset{\displaystyle CH_3}{CH}CH_2O)_2R_8$$

où $R_8$ est $CH_3$, —$CH_2CH_3$; —$CH(CH_3)_2$ ou —$CH_2CH_2Cl$.

4. Un composé selon la revendication 3, dans lequel $R_2$ est H, Cl ou $CH_3$ et R est un radical alcoyle en $C_1$—$C_4$; alcényle en $C_3$—$C_4$; alcoyle en $C_2$—$C_3$ substitué par —$OCH_3$ ou Cl; alcényle en $C_3$ substitué par 1—3 atomes de Cl; cycloalcoyle en $C_5$—$C_6$; cyclohexényle; cyclohexyle substitué par 1—3 groupes méthyle; —$CH_2CH_2OR_7$ où $R_7$ est —$C_2H_5$, —$CH(CH_3)_2$, un radical phényle ou —$CH_2CH_2Cl$; ou

$—CHCH_2OC_2H_5.$
|
$CH_3$

5. Un composé selon la revendication 4, dans lequel $R_2$ et $R_3$ sont tous deux H.

6. Un composé selon la revendication 3, 4 ou 5, dans lequel R est un radical alcoyle en $C_1—C_4$; alcoyle en $C_2—C_3$ substitué par Cl; alcényle en $C_3—C_4$; $—CH_2CH_2OCH_3$;

$—CHCH_2OCH_3$; $—CH_2CH_2OC_2H_5$; $—CHCH_2OC_2H_5$;
|  |
$CH_3$  $CH_3$

ou $—CH_2CH_2CH_2OC_2H_5$.

7. Le 2-(isocyanatosulfonyl)benzoate de méthyle.

8. Le 2-(isocyanatosulfonyl)benzoate d'isopropyle.

9. Le 2-(isocyanatosulfonyl)benzoate d'allyle.

10. Le 2-(isocyanatosulfonyl)benzoate de 1-(2-chloroéthyle).

11. Le 2-(isocyanatosulfonyl) benzoate de propyle.

12. Un procédé pour préparer un composé tel que défini dans la revendication 1, selon lequel on fait réagir un composé de formule générale

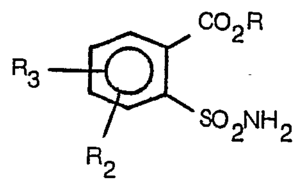

dans laquelle R, $R_2$ et $R_3$ sont tels que définis dans la revendication 1, avec du phosgène en présence d'une quantité catalytique d'un isocyanate d'alcoyle et d'une quantité catalytique de 1,4-diaza[2,2,2]-bicyclooctane dans un solvant inerte.